# EUROPÄISCHE PATENTSCHRIFT

(11) **EP 0 714 267 B1**
(45) Veröffentlichungstag und Bekanntmachung des Hinweises auf die Patenterteilung: **03.05.2000**
(21) Anmeldenummer: 94925464.3
(22) Anmeldetag: 19.08.1994
(51) Int. Cl.: A61B 10/00, A61B 17/20

(54) **VORRICHTUNG ZUM PARTIELLEN KENNZEICHNEN, BESCHÄDIGEN UND BENETZEN EINER OBERFLÄCHE BEI DER DURCHFÜHRUNG EINES ALLERGIETESTS**
DEVICE FOR PARTIALLY MARKING, INJURING AND WETTING A SURFACE DURING ALLERGEN TESTING
DISPOSITIF D'IDENTIFICATION PARTIELLE, D'ENDOMMAGEMENT ET DE MOUILLAGE D'UNE SURFACE POUR TESTS D'ALLERGIES

(30) Priorität: 20.08.1993 DE 4328112
(43) Veröffentlichungstag der Anmeldung: 05.06.1996
(73) Patentinhaber: Schindlbeck, Karin, D-68519 Viernheim (DE)
(72) Erfinder: Schindlbeck, Karin, D-68519 Viernheim (DE)
(74) Vertreter: Alber, Norbert, Dipl.-Ing.
(86) Internationale Anmeldenummer: EP9402771
(87) Internationale Veröffentlichungsnummer: WO9505776

(56) Entgegenhaltungen:
- EP-A- 0 006 158
- EP-A- 0 026 501
- EP-A- 0 081 975
- EP-A- 0 211 312
- EP-A- 0 335 231
- WO-A-87/05200
- WO-A-88/09149
- US-A- 2 817 336
- US-A- 5 104 620

## Beschreibung

Die Erfindung betrifft eine Vorrichtung, mittels welcher eine Oberfläche in Teilbereichen gekennzeichnet, beschädigt und mit einer Flüssigkeit benetzt werden kann. Diese Vorgehensweise wird in großem Stil bei Allergietests an Patienten angewandt, wobei die Haut des Patienten durch Ritzen oder Anstechen beschädigt und unter Einwirkung eines Allergens gebracht werden muß, um die Wirkung des Allergens auf den Patienten zu testen. Da dies parallel für eine Vielzahl von Allergenen vorgenommen wird, müssen viele Einzelbereiche der Haut, beispielsweise entlang des Unterarmes eines Patienten auf diese Art und Weise bearbeitet werden.

Bei derartigen Allergietests ist es bisher üblich, die Haut des Patienten, beispielsweise entlang des Unterarmes, mittels eines Markierungsstiftes in eine Vielzahl von zur Unterscheidung per Hand numerierten Feldern einzuteilen, die entsprechende Hautfläche waagrecht zu lagern, aus einem Sortimentkasten, der einzelne Tropfer-Fläschchen für die Allergene enthält, die einzelnen Fläschchen zu entnehmen und auf die markierten Felder der Haut des Patienten einen Tropfen der entsprechenden Flüssigkeit aufzubringen.

Dabei muß die Numerierung des Feldes mit der ebenfalls vorhandenen Numerierung der Allergen-Fläschchen übereinstimmen, da ansonsten das Testergebnis falsch interpretiert würde.

Es muß ferner darauf geachtet werden, daß die Flüssigkeiten der einzelnen Felder nicht ineinanderlaufen, und anschließend - nach Aufbringen aller Allergene auf der Hautoberfläche - wird durch jeden einzelnen Flüssigkeitstropfen hindurch die Hautoberfläche des Patienten mit einer Nadel oder Ähnlichem kurz angestochen. Zwischen den einzelnen Stichen muß die Nadel etc. jeweils sorgfältig gereinigt werden, um die gebrauchten, flüssigen Allergene nicht untereinander zu vermischen.

Anschließend müssen die einzelnen Allergene eine ausreichende Zeit auf die beschädigte Hautoberfläche einwirken können, um dann eine Rötung der Haut in diesem Bereich positiv oder negativ feststellen zu können, die für den Arzt den Rückschluß auf eine Überreaktion des Patienten auf bestimmte Substanzen zuläßt.

Diese Methode weist neben dem hohen Zeitaufwand vier weitere Nachteile auf, insbesondere mehrere Fehlermöglichkeiten bei der Durchführung des Tests etwa durch Verlaufen der Flüssigkeiten, Vermischen der Allergene beim Anstechen der Haut, nicht ausreichendes Beschädigen der Haut, falsche Zuordnung der Allergennumerierung zur Flächennumerierung.

Ein weiterer Nachteil liegt darin, daß die Allergene in Fläschchen bevorratet werden, die die Menge für eine Vielzahl von Allergietests enthalten, jedoch auf der anderen Seite nur begrenzt haltbar sind. Aus diesem Grund wird ein Arzt nur dann einen solchen Allergietest zur Verfügung halten, wenn er mit einem gewissen Mindestumsatz an Testpersonen rechnen kann.

Aus der US 5,104,620 ist eine Allergietestvorrichtung bekannt, die eine obere Schicht aufweist, in der Öffnungen vorgesehen sind, die Druckknöpfe aufnehmen, an deren Unterseite jeweils eine Nadel angeordnet ist. Unter Einhaltung eines gewissen Abstandes ist unterhalb der oberen Schicht eine Membranschicht angeordnet, in der Kammern ausgebildet sind, die ein Antigen aufnehmen. Die Kammern werden mittels einer entfernbaren Schutzschicht verschlossen. Da bei dieser vorbekannten Vorrichtung die obere Schicht und die Membranschicht zusammen ein Teil bilden, ist eine vergleichsweise hohe Biegesteifigkeit gegeben, die sich nachteilig auf die Handhabung an der gekrümmten Hautoberfläche eines Patienten auswirkt.

Aus der EP-A-0 081 975 ist eine Allergietestvorrichtung bekannt, die aus einen flexiblen Klebeband besteht, das an seiner klebenden Seite wenigstens eine in die Haut eindringende Nadel, die ein Allergen trägt, aufweist. Zur Anwendung dieser Vorrichtung wird eine die Klebeseite des Klebebandes sowie die Nadel schützende Schicht abgezogen und daraufhin das Klebeband auf die gewünschte Hautoberfläche des Patienten geklebt. Dabei kann es vorkommen, daß sich das Klebeband aufgrund seiner zu geringen Biegesteifigkeit in sich verklebt und somit die Handhabung erschwert wird.

Des weiteren ist aus der WO-A-88 09 149 eine medizinische Testvorrichtung bekannt, die aus einer mit der Hautoberfläche in Kontakt kommenden Abdeckung mit in parallelen Reihen nebeneinander angeordneten Durchbrüchen sowie einer Behältereinheit besteht. Die Behältereinheit weist mehrere zur gleichen Seite hin offene und mit den Durchbrüchen korrespondierende Behälter auf, in denen sich die Allergene befinden. Abdeckung und Behältereinheit sind mittels eines Klebstoffes miteinander verbunden, so daß diese Vorrichtung immer in diesem geschichteten Zustand verwendet wird. Dies hat zur Folge, daß hier die Biegesteifigkeit der Vorrichtung relativ hoch ist, so daß das Auflegen der Vorrichtung auf gekrümmte Oberflächen nur durch Inkaufnahme einer schlechteren Isolierung der einzelnen Teststellen voneinander möglich ist. Darüber hinaus ist es auch bei dieser Vorrichtung nicht möglich, die einzelnen Hautteststellen optisch zu überwachen sowie individuell voneinander getrennt vorzubereiten und zu behandeln.

Es ist daher die Aufgabe der Erfindung, eine Vorrichtung zum partiellen Kennzeichnen, Beschädigen und Benetzen einer Oberfläche bei der Durchführung eines Allergietests bei der die Teststellen der Oberfläche individuell sichtbar und bearbeitbar sind, die Vorrichtung eine die Handhabung vereinfachende Biegesteifigkeit aufweist, so daß die Haftung auf der gekrümmten Oberfläche möglichst dauerhaft ist, und die Fehlerwahrscheinlichkeit beim Anwenden der Vorrichtung möglichst gering ist.

Diese Aufgabe wird mit Hilfe einer Vorrichtung mit den Anspruchs 1 gelöst. Vorteilhafte Ausführungsformen ergeben sich aus den Unteransprüchen.

Dabei erfolgt die Einteilung der Bereiche der Oberfläche sowie deren Kennzeichnung nicht direkt auf der Haut, sondern durch Auflegen einer Abdeckung, die eine Reihe von Durchbrüchen, beispielsweise in zwei parallelen Reihen nebeneinander angeordnet, aufweist, die jeweils direkt auf der Abdeckung numeriert sind.

Die Befestigung dieser Abdeckung kann entweder mittels um den Unterarm herumgeschlungener Klettbänder oder auch mittels Haftung auf der Haut des Patienten erfolgen. Dies ist entweder mittels bekannter Klebstoffe, wie sie auch bei Heftpflastern etc. verwendet werden, möglich, was jedoch die Gefahr in sich birgt, daß der Patient gerade auf diesen Klebstoff allergisch reagiert, und dadurch das Testergebnis verfälscht wird. Eine andere Möglichkeit besteht darin, wenigstens die der Haut zugewandte Seite der Abdeckung aus einem klebstofffreien, aber dennoch selbsthaftenden Kunststoff auszubilden, dessen Haftung ausschließlich auf der hohen Flexibilität dieses Kunststoffes und der engen Anlagerung an die Haut basiert.

Derartige Kunststoffe sind an sich bekannt, sind jedoch in ihrer Handhabung ohne Stütz- und Trägerfolie sehr schwierig, da ihre sehr hohe Flexibilität und Haftung an Oberflächen sehr leicht zu einem Zusammenkleben in sich selbst führt.

Eine solche selbsthaftende Folie könnte jedoch auf der eigentlichen Abdeckung selbst mittels Kleben aufgebracht sein, und müßte dabei nicht unbedingt die gesamte Fläche der Abdeckung umfassen, sondern es wäre bereits der Ringbereich um die einzelnen Durchbrüche herum ausreichend.

Eine solche selbsthaftende Folie müßte bis zum Gebrauch durch eine übergelegte Schutzfolie vor Kontakt mit anderen Oberflächen geschützt werden und eventuell auch separat, ohne vorherige Verbindung mit der Abdeckung, auf der Haut des Patienten aufgebracht werden, um diese vor der Einwirkung von Klebstoffen zu schützen, mit deren Hilfe die Abdeckung haften soll.

Nach dem Befestigen der Abdeckung kann die Hautoberfläche beschädigt werden, in dem mit einer Nadel oder einem anderen spitzen Gegenstand entlang der Reihen der Durchbrüche, die anschließend als Arbeitsfelder dienen jeweils durchgehend geritzt wird, wobei die Haut zwischen den einzelnen Arbeitsfeldern durch die Abdeckung vor dem nicht notwendigen Ritzen geschützt wird. Aus diesem Grund muß die Abdeckung aus einem ausreichend ritzfesten bzw. festen und elastischen, stabilen Kunststoff bestehen. Auch das Befestigen der Abdeckung muß eine ausreichend feste Verbindung zur Haut gewährleisten, damit keine Verschiebung der Abdeckung durch das Ritzen eintreten kann.

Nach dem Unterteilen, Kennzeichnen und Beschädigen der Hautoberfläche werden die Allergene nicht einzeln auf die Arbeitsfelder aufgeträufelt, sondern es wird eine Behältereinheit direkt auf die Abdeckung aufgelegt, in der sich einseitig offene Behälter mit den festen oder flüssigen Allergenen in einer solchen Anordnung befinden, daß diese mit den Durchbrüchen der Abdeckung, also den Arbeitsfeldern, fluchten. Da zur leichteren Handhabung bei diesem Vorgang der bearbeitete Hautbereich in der Regel etwa waagrecht liegen wird, muß diese Behältereinheit beim Auflegen auf die Abdeckung gestürzt werden, so daß die flüssigen Allergene nicht in einseitig offenen Vertiefungen des Behälters vorliegen dürfen, da sie sonst beim Stürzen herauslaufen, sich vermischen und ähnliche unerwünschte Effekte auftreten würden.

Daher sind die einzelnen Behälter der Behältereinheit beim Stürzen und Auflegen auf die Abdeckung noch verschlossen und werden erst nach dem Auflegen, und damit ohne erneutes Abheben gegenüber den Arbeitsfeldern, geöffnet. In diesem Fall kann das Allergen als Flüssigkeit innerhalb des Behälters vorliegen. Das Öffnen kann beispielsweise durch Hindurchstechen von der Rückseite der aufgelegten Behältereinheit her erfolgen. Dies ist möglich durch eine Verletzungseinheit, also eine Art Nadelkissen, welches im Bereich jedes Arbeitsfeldes eine Nadel oder einen spitzen Gegenstand aufweist, und separat nach dem Auflegen der Behältereinheit auf die Abdeckung auf die Rückseite der Behältereinheit aufgelegt und angedrückt wird.

Die Nadeln können dabei auch auf den Boden jeder Vertiefung angeordnet sein, so daß durch Aufdrücken auf die Rückseite der einzelnen Behälter der Behältereinheit, z. B. mittels einer stabilen Drückplatte, die mit dem Wirkstoff benetzten Nadeln in die Oberfläche eingedrückt werden. Die Nadeln können hierbei auch als mit Allergen trocken geimpfte Spitzen ausgeführt werden, so daß keine Folie mehr an der Behältereinheit erforderlich ist.

Als Behältereinheit kann auch eine sogenannte Hohlkammerfolie benutzt werden, die aus zwei parallel liegenden Folien beteht, die netzwerkartig miteinander verschweißt werden, so daß dazwischen gegeneinander abgeschlossene Hohlräume entstehen. Die dabei verwendeten Folien sind relativ dünn und flexibel, so daß vor dem Verkleben der beiden Folien miteinander eine der Folien in einen geformten Untergrund mit entsprechenden Vertiefungen eingelegt und die einzelnen flüssigen Allergene in diese Vertiefungen eingebracht werden könnten.

Ebenso kann in eine mit Vertiefungen ausgestattete Behältereinheit in jede Vertiefung eine geschlossene Kapsel eingelegt werden, die den jeweiligen Wirkstoff, vorzugsweise in flüssiger Form, enthält. Die Kapsel wird in der Vertiefung entweder durch einen in der Vertiefung vorhandenen Klebepunkt gehalten oder durch einen Hinterschnitt der Vertiefung, also einen verengten Hals.

Durch das Einsetzen derartiger einzelner Kapseln kann eine individuelle Bestückung der Behältereinheit vor dessen Anwendung erzielt werden.

Eine andere Lösung bei dieser Vorgehensweise besteht darin, die Nadeln oder Spitzen zum Öffnen der bereits aufgelegten Behälter bereits innerhalb der Behälter, also beispielsweise auf dem Boden der einzelnen Behälter anzubringen. Die Spitzen müssen dabei jedoch so kurz sein, daß sie im normalen Zustand die abdeckende Stützfolie nicht durchstoßen, sondern dies nur nach einem Anpressen des aufgelegten Behälters von der Rückseite her möglich ist.

Als Spitzen können neben mechanisch festen Spitzen wie Metallspitzen etc. auch harte Fasern oder Bündel von Fasern wie etwa Glasfasern oder harte, spitze Kunststoffasern als Wirkstoffträger dienen, wobei vor allem Faserbündel zum Handhaben von trockenen Wirkstoffen geeignet sind, die auf die relativ große Oberfläche der Faserbündel-Spitzen gut aufgebracht werden können.

Zu diesem Zweck muß auch das Material, aus welchem die Behältereinheit selbst, insbesondere die darin als Vertiefungen eingeformten Behälter, besteht, ausreichend stabil sein, um nicht bereits bei einer leichten Berührung sich soweit zu verformen, daß die eingebrachte Spitze die gegenüberliegende Abdeckfolie durchstößt und damit der Behälter vorzeitg undicht wird.

Die andere Vorgehensweise besteht darin, die Allergene nicht als lose Flüssigkeiten in die Behälter einzubringen, sondern sie darin so unterzubringen, daß auch nach Öffnen der Oberseite der Behälter, beispielsweise durch Abziehen einer durchgehenden Schutzfolie, ein Stürzen der Behälter auf die Abdeckung möglich ist, ohne daß die Allergene unerwünscht entweichen.

Dies ist möglich, indem innerhalb jedes Behälters ein Vlies oder ein anderes schwammartiges Gewebe eingebracht ist, in welchem die flüssigen Allergene aufgesaugt sind, und zwar in einer solchen Menge, daß beim einfachen Stürzen noch kein Heraustropfen der Allergene erfolgt, jedoch beim Andrücken der aufgelegten Behälter von der Rückseite her die Flüssigkeit gegen die zu beaufschlagende Hautoberfläche herausgedrückt und diese angefeuchtet wird.

Eine andere Möglichkeit besteht darin, die Allergene nicht als Flüssigkeiten, sondern in Form eines Gels in den Behältern aufzunehmen, welches ausreichend pastös ist, um beim Stürzen des Behälters nicht herauszutropfen. Auch in diesem Fall wird das Gel durch rückseitigen Druck auf den aufgelegten Behälter bzw. die ganze Behältereinheit in Kontakt mit der Hautoberfläche gebracht. Ein weiterer Vorteil dieser Lösung besteht darin, daß ein Gel im Gegensatz zu einer Flüssigkeit eine geringere oder überhaupt keine Kapillarwirkung zeigt und damit ein Unterlaufen der Abdeckung von einem Arbeitsfeld zum anderen hin mittels ausreichender Haftung zwischen Abdeckung und Hautoberfläche nicht vorkommen kann und damit auf entsprechende Gegenmaßnahmen bei der Gestaltung der Abdeckung ebenfalls verzichtet werden kann.

Der rückseitige Druck auf die aufgelegte Behältereinheit geschieht durch Aufpressen mittels einem entsprechenden Gegenstand, beispielsweise einer Platte, oder Überrollen mittels einer Gummiwalze oder einem ähnlichen Gegenstand. Falls das Aufpressen über einen längeren Zeitraum gewünscht wird, kann dies auch mittels der bei jedem Arzt vorhandenen Blutdruckmanschette oder einer eng aufgebrachten Bandage aus Klettband etc. erfolgen.

Bei der Gestaltung und Positionierung der Durchbrüche als Arbeitsfelder in der Abdeckung einerseits sowie der Behälter in der Behältereinheit andererseits ist darauf zu achten, daß beim Auflegen die Durchbrüche und die Behälter fluchten. Die Abdeckung ist daher mit der Behältereinheit verbunden.

So könnten Behältereinheit und Abdeckung mittels einer klappbaren, als Scharnier ausgebildeten, Verbindung einstückig hergestellt sein, und nach dem Auflegen und Befestigen der Abdeckung, Entfernen einer Schutzfolie von der Seite der Behältereinheit, in welcher die offene Seite der einzelnen Behälter liegt, wird die Behältereinheit auf die Abdeckung aufgeklappt und dort vor dem Anpressen zum Schutz vor Verschieben auch fixiert werden.

Das Fixieren kann durch partielles Verkleben mit der Abdeckung erfolgen, oder durch separates Fixieren mittels Klettband-Streifen um den Arm des Patienten herum.

Bei der Verwendung von flüssigen Allergenen, die in einem Vlies oder Schwämmchen in den einzelnen Behältern aufgenommen sind, kann unter Verlängerung der Einwirkungszeit auch ganz auf das Anpressen der Behältereinheit verzichtet werden, sofern der Arm des Patienten etwa waagrecht gelagert wird. Denn bei dieser Vorgehensweise wird allein aufgrund der Schwerkraft das flüssige Allergen durch das Vlies oder Schwämmchen hindurch nach unten sickern und nach einer gewissen Zeit auch die Unterseite des Schwämmchens feucht sein. Falls dieses Schwämmchen so dimensioniert oder eingesetzt ist, daß es sich bei abgezogener Schutzfolie, also ohne Gegendruck, aus der Oberseite der als Halterung dienenden Vertiefung hervorwölbt, wird dieses Vlies oder Schwämmchen bei richtig aufgelegter Behältereinheit in Kontakt mit der Hautoberfläche stehen und damit auch ohne Andrücken nach ca. 10 bis 30 Sekunden ein Kontakt der Flüssigkeit mit der geritzten Hautoberfläche zustandekommen.

Das Einbringen solcher Schwämmchen oder Vliesstücke in die Behälter, also vorzugsweise in Vertiefungen der Behältereinheit, soll ein späteres Herausfallen zuverlässig verhindern. Da ein Einkleben mittels Klebstoff aufgrund möglicher Verfälschungen der Allergietests unerwünscht ist, wird vorzugsweise für die Behälter ein Kunststoff verwendet, der durch ein Lösungsmittel angelöst werden kann, während für das Vlies oder Schwämmchen ein Material verwendet wird, welches durch dasselbe Lösungsmittel nicht geschädigt wird. Dadurch ist es möglich, beim Einsetzen der Schwämmchen oder Vliesstücke diese mit dem betreffenden Lösungsmittel in einer solchen Menge und Konzentration zu tränken, daß nach dem Einsetzen die kontaktierten Bereiche innerhalb der Vertiefungen oberflächlich durch das Lösungsmittel angelöst, jedoch nicht durchbrochen werden. Aufgrund der Flüchtigkeit der Lösungsmittel, beispielsweise der leichten Flüchtigkeit von Aceton, wird diese oberflächliche Anlösung schnell wieder zu einer Verfestigung führen, wobei die während dieses Vorganges an die Vertiefungswandungen gedrückten Bereiche der Schwämmchen dort oberflächennah geringfügig in die Behälteroberfläche sozusagen eingegossen werden und damit formschlüssig verhaftet sind.

Die Vlies-Stücke können in den Vertiefungen aber auch mechanisch gehalten werden, in dem die Vertiefungen eine entsprechende Hinterschneidung, also in der Nähe ihrer offenen Seite eine halsartige Verengung, besitzen. Wenn diese vor Einsetzen des Vlieses hergestellt wird, muß das Vlies durch diese Verengung wenigstens teilweise hindurchgedrückt werden, um sich dahinter ausdehnen und einen ausreichenden mechanischen Rückhalt gewährleisten zu können.

Ebenso kann die Verengung jedoch auch erst nach Einlegen des Vlieses hergestellt werden, indem der Kunststoff im Bereich der Seitenwände der Vertiefungen, also des späteren Halses, Schrumpfeigenschaften aufweist bzw. mit einer die entsprechende Schrumpfeigenschaft besitzenden Folie belegt ist. Durch Erwärmen dieser Seitenwände wird der Hals geschrumpft und dadurch das Vlies in der Vertiefung gehalten.

Falls nach dem Aufbringen der Wirkstoffe auf die Oberfläche die Abdeckung - beispielsweise aus allergetischen Gründen - nicht mehr auf der Oberfläche aufliegen soll oder auf eine solche Abdeckung vollständig verzichtet werden soll, ist es möglich, eine Kennzeichnung beispielsweise in Form einer Numerierung automatisch auf der Oberfläche in der Nähe jedes Arbeitsfeldes aufzubringen:

Zu diesem Zweck ist das auf der Oberfläche aufzulegende Teil, also entweder die Behältereinheit oder die Verletzungseinheit, mit einer übergabefähigen Kennzeichnung ausgestattet, also entweder einer Kennzeichnung mittels übertragbarer und auf der gewünschten Oberfläche gut haftender Farbe oder einer übertragenden, kennzeichnenden Folie ähnlich einem Abziehbild. Die Haftung gegenüber der Oberfläche muß dabei so eingestellt sein, daß durch einfaches Andrücken der entsprechenden Einheit auf der Oberfläche die Kennzeichnung auf die Oberfläche wenigstens teilweise und damit erkennbar übergeht.

Einzelne Ausführungsformen gemäß der Erfindung sind im Folgenden anhand der Figuren beispielhaft näher beschrieben.
Es zeigen:
Figur 1 eine perspektivische Ansicht einer gesamten Vorrichtung,
Figur 2a eine Detaildarstellung einer alternativen Ausführungsform anhand von nur einem Behälter in unbenutztem Zustand (Verpackungszustand),
Figur 2b eine Detaildarstellung gemäß Figur 2a in aufgeklapptem Zustand beim Abziehen der Schutzfolie ,
Figur 2c eine Detaildarstellung gemäß Figur 2a im auf die Oberfläche aufgelegten Zustand beim Andrücken der Nadeln mit Hilfe einer Pressplatte,
Figur 3 eine gegenüber Figur 1 erweiterte Vorrichtung,
Figur 4 eine Behältereinheit mit einer Hohl-Folie
Figur 5a einen mit einem Vlies gefüllten Behälter im Verpakkungszustand,
Figur 5b einen mit einem Vlies gefüllten Behälter mit abgezogener Schutzfolie.

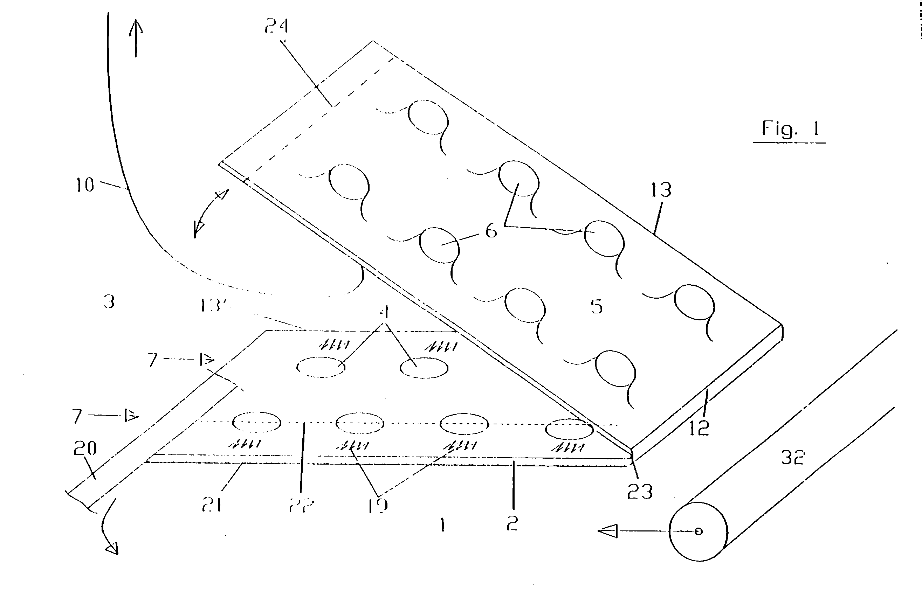

Figur 1 zeigt eine gesamte Vorrichtung, bestehend aus einer Abdeckung 2 und einer Behältereinheit 5, die an ihrer Schmalseite, der Kante 12, durch dort dünner ausgebildetes Material als Scharnier 23 gegeneinander klappbar ausgebildet sind. Die Abdeckung 2 und die Behältereinheit 5 weisen dabei identische Außenkonturen 13, 13' auf, und liegen beim Aufeinanderklappen fluchtend aufeinander.

Die Abdeckung 2 ist dabei entweder mittels des Klebers 21 auf der darunter befindlichen Hautoberfläche 1 befestigt, oder mittels wenigstens zweier, um den Unterarm oder den entsprechenden Körperteil des Patienten herumgewickelter Klettbänder 20, die fest mit der Abdeckung 2 verbunden sind und von denen in Figur 1 nur am linken vorderen Eck der Abdeckung 2 eines dargestellt ist.

In Figur 1 ist auch die Ritzung 22 zu erkennen, die mittels einer Nadelspitze oder eines spitzen Gegenstandes über die als Arbeitsfelder 4 dienenden Durchbrüche 3 hinweggeführt wurde und somit eine Ritzung einerseits auf der innerhalb der Arbeitsfelder 4 befindlichen Hautpartien als auch auf der zwischen den Durchbrüchen befindlichen Abdeckung 2 hinterläßt. Der Vorteil besteht im schnellen und effizienten Einbringen der Ritzung, wobei für jede Reihe 7 von Durchbrüchen 3 nur jeweils eine schnelle Ritzung erfolgen muß.

In Figur 1 ist ferner neben jedem Durchbruch 3 die nicht entfernbare Numerierung 19 zu erkennen, anhand derer nach Beobachtung der Rötung das zugeordnete Allergen bestimmbar ist.

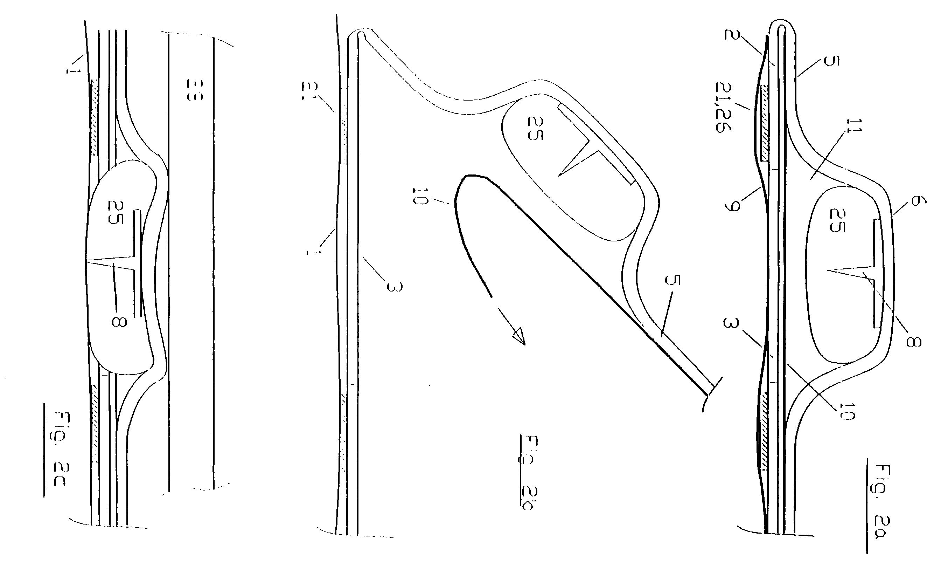

In Figur 1 befindet sich die Behältereinheit 5, von der zuvor die Schutzfolie 10 abgezogen wurde, um die Öffnungen der einzelnen Behälter 6 auf der zur Abdeckung 2 hin gerichteten Seite offenzulegen, im Abklappen in Richtung der Abdeckung 2. An dieser wird die Behältereinheit 5 vorzugsweise mittels eines Klebestreifens 24, der sich jenseits am freien Ende der Behältereinheit 5 befindet, fixiert. Anschließend erfolgt das Andrücken der Behälter 6 von deren Rückseite her durch Überrollen mittels einer Rolle 32 (oder Anpressen mittels einer Pressplatte, wie in Figur 2c dargestellt), um den Inhalt der Behälter 6 in Kontakt mit der Oberfläche 1 zu bringen.

Die Figuren 2a bis 2c zeigen anhand von nur einem der Behälter 6 einer Behältereinheit 5 eine andere Ausbildung der Vorrichtung im Detail:

Im verpackten Zustand sind die Abdeckung 2 und die Behältereinheit 5 zur raumsparenden Aufbewahrung ebenfalls in Parallellage aneinandergeklappt. Die Schutzfolie 10 deckt die offenen Seiten der von den Behältern 6 geformten Hohlräume 11 der Behältereinheit 5 ab. Im Inneren jedes Hohlraumes 11 befindet sich auf dem Boden des napfförmigen Behälters 6, also in der in den Figuren 2a bis 2c dargestellten Lage an der inneren Oberseite, eine Spitze 8, deren freies Ende jedoch in diesem Zustand ausreichenden Abstand von der Schutzfolie 10 einhält.

Auf der in Figur 2a unteren Seite der Abdeckung 2 befindet sich um die Durchbrüche 3 herum ein umlaufender Bereich der - je nach Empfindlichkeit der Patienten - entweder aus einen Kleber 21 oder einer selbsthaftenden Folie 26 besteht und in verpackten Zustand von einer Abdeckfolie 9 geschützt ist.

Nachdem diese Abdeckfolie 9 entfernt ist, kann die Abdeckung 2 auf der zu bearbeitenden Oberfläche 1 aufgelegt, angedrückt und somit fixiert werden. Nach Abziehen der Schutzfolie 10 der Behältereinheit 5, wie in Figur 2b dargestellt, wird die Behältereinheit 5 gemäß Figur 2c auf die Abdeckung aufgelegt und gegen die Oberfläche 1 mittels einer Pressplatte 28 oder anderweitig aufgepresst. Dadurch wird die Nadel 8 durch den Durchbruch 3 der Abdeckung 2 hindurch in die Oberfläche 1 der Haut gedrückt und beschädigt diese, die gleichzeitig mit dem das Allergen enthaltenden Gel 25, welches im Behälter 6 aufgenommen war, in Berührung kommt.

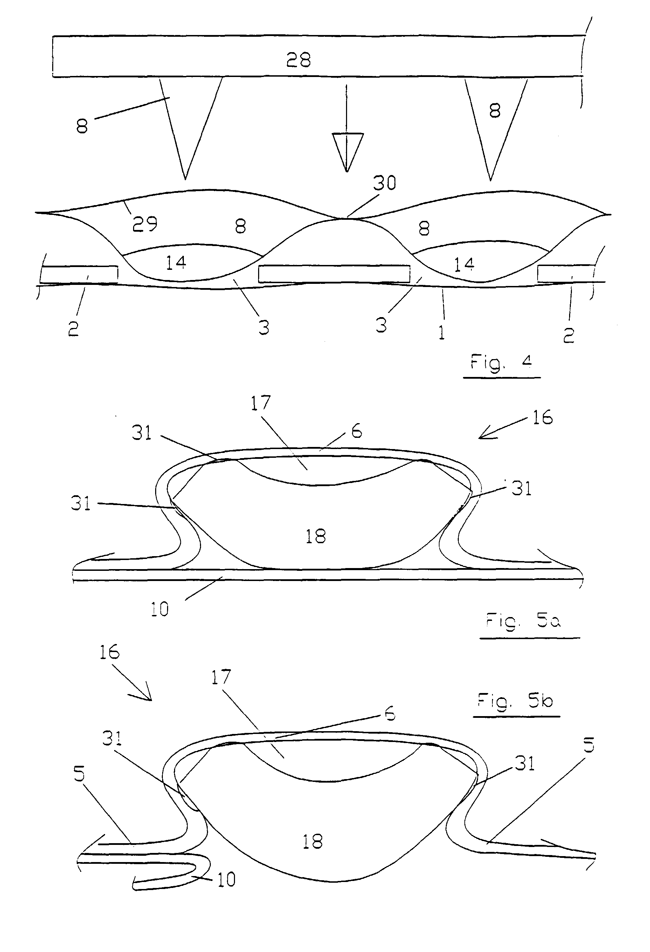

Anstelle eines formbeständigen Behälters 6 bzw. einer ganzen formbeständigen Behältereinheit 5, wie in den Figuren 1 und 2a bis 2c dargestellt, kann als Behältereinheit 5 auch eine relativ instabile Hohlkammerfolie 29 verwendet werden, wie in Figur 4 dargestellt. Diese wird nach Befestigung der Abdeckung 2 ebenfalls passend auf die Abdeckung 2 aufgelegt, und anschließend mittels einer Pressplatte 28 gegen die Oberfläche 1 gedrückt, wobei sich Nadeln 8 in den den Durchbrüchen 3 entsprechenden Positionen auf der Unterseite der Pressplatte 28 befinden und beide Folien der Hohlkammerfolie sowie die Hautoberfläche 1 durchstoßen. Wenn die Pressplatte 28 ausreichend lange Zeit, wenigstens einige Sekunden lang, unter Druck aufgelegt bleibt, wird dadurch die die Allergene enthaltende Flüssigkeit 14 durch die entstandene Öffnung der unteren Folie der Hohlkammerfolie 29 herausgedrückt und in Kontakt mit der beschädigten Oberfläche 1 geraten.

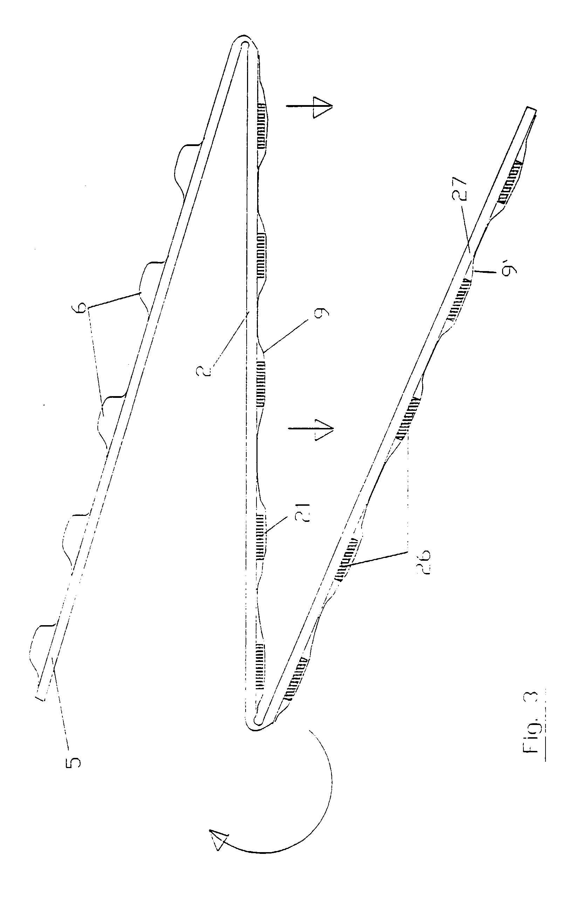

Eine etwas aufwendigere, jedoch sehr zuverlässig und schonend arbeitende Vorrichtung in Erweiterung der Lösung gemäß Figur 1 zeigt Figur 3.

Dabei ist am freien Ende der Behältereinheit 5 eine zusätzliche, mit gleichen Abmessungen und Konturen gestaltete, Stützfolie 27 gelenkig befestigt. Nach Parallellage dieser Stützfolie 27 befinden sich an deren Unterseite wenigstens um die Bereiche, die der Umgebung der Durchbrüche 3 der Abdeckung 2 entsprechen, diese Durchbrüche 3 umschließende, alleine nicht handhabbare selbsthaftende Folien 26, die zunächst noch mit einer Abdeckfolie 9' abgedeckt sind. Nach Abziehen dieser Abdeckfolien 9' und Aufpressen der Stützfolie 27 auf die Haut bleiben diese selbsthaftenden Folien 26 auf der Haut kleben, gegenüber welcher sie eine höhere Haftung besitzen als gegenüber der relativ glatten Stützfolie 27. Die Stützfolie 27 wird daraufhin von der Haut des Patienten entfernt und in eine Ebene mit der Abdeckung 2 gebracht, oder von dieser durch Abriß mittels Perforierung an der Verbindungskante abgetrennt und weggeworfen. Anschließend wird die Behältereinheit 5 auf die mit selbsthaftender Folie 26 belegten Bereiche der Haut so aufgelegt, daß die auf der Unterseite der Abdeckung 2 angeordneten Kleber 21 auf den auf der Haut befindlichen selbsthaftenden Folien 26 zu liegen kommen und dort die Behältereinheit 5 fest verkleben.

Dadurch kommt die Haut nicht direkt mit dem Kleber in Kontakt, sondern lediglich mit der selbsthaftenden Folie 26, die weder Lösungsmittel noch Kleber enthält, und somit selbst keine Allergien auslösen kann und auch das Testergebnis nicht verfälschen kann, und darüberhinaus beim Abziehen von der Hautoberfläche keinerlei Schmerz verursacht. Anschließend wird wie vorher beschrieben vorgegangen.

Die Figuren 5a und 5b zeigen eine Detaildarstellung der Anordnung eines Vlieses 18 innerhalb der Behälter 6 einer Behältereinheit 5, die als Blisterpackung ausgeführt sein kann. Eine besonders einfache Befestigung des Vlieses 18 ist gegeben, wenn der als Behälter dienende Hohlraum 11 einen Hinterschnitt aufweist, in den sich das Vlies 18 einspreizen kann.

Da eine Herstellung solcher Vertiefungen jedoch schwierig ist, genügt auch eine Ausbildung der Vertiefungen der Behälter 6 mit etwa parallelen, senkrecht zur Oberfläche verlaufenden Wänden. In einen solchen Behälter kann das im entspannten Zustand plattenförmige Vlies 18, welches eine größere Abmessung besitzt als die Grundfläche des Behälters 6, konkav gewölbt eingesetzt werden, wodurch es sich mit seinen Außenkanten gegen die Wände der Vertiefung des Behälters 6 spreizen wird. Wenn bei Einsetzen das Vlies 18 mit Aceton oder einem anderen Lösungsmittel getränkt ist, und der Kunststoff des Behälters 6 von diesem Lösungsmittel angelöst werden kann, wird in den Kontaktflächen zwischen Vlies 18 und dem Behälter 6 ein Bereich 31 zunächst angelöst und bei Verdunstung des Lösungsmittels wieder verfestigt, wobei während dieser Verfestigung die sich durch die Eigenspannung des gewölbten Vlieses 18 anpressenden Bereiche miteingebunden und mechanisch festgehalten werden.

Dabei ist es wichtig, daß das Vlies 18 so groß dimensioniert ist, daß es sich ohne Gegendruck, also beispielsweise einer übergeklebten Schutzfolie 10 aus dem Behälter 6 herauswölbt, wodurch sichergestellt ist, daß bei aufgelegter Behältereinheit 5 auf die Abdeckung 2 das Vlies 18 in Kontakt mit der Haut gerät, indem es gegenüber dem Boden des Behälters 6 konvex gewölbt eingebracht ist und sich damit im mittleren Bereich nach außen wölbt.

## Patentansprüche

1. Vorrichtung zum partiellen Kennzeichnen, Beschädigen und Benetzen einer Oberfläche bei der Durchführung eines Allergietests, die
- eine Abdeckung (2) aus Kunststoff, in der in etwa parallelen Reihen nebeneinander Durchbrüche (3) angeordnet sind, sowie
- eine Behältereinheit (5), deren Grundfläche im wesentlichen der der Abdeckung (2) entspricht und die eine im wesentlichen flache Unterseite aufweist, beinhaltet, wobei
- in der Behältereinheit (5) mit den Durchbrüchen (3) korrespondierende Behälter (6) vorgesehen sind, die alle einseitig zur gleichen Seite hin offen sind und jeweils als Reservoir für Wirkstoffe fungieren,
**dadurch gekennzeichnet, daß**
- die Abdeckung (2) und die Behältereinheit (5) an einer Kante (12) klappbar miteinander verbunden sind, so daß die Behälter (6) der Behältereinheit (5) auf die Durchbrüche (3) der Abdeckung (2) fluchtend aufgelegt werden können, und
- die Oberfläche durch Zusammenklappen von Abdeckung (2) und Behältereinheit (5) benetzbar ist.

2. Vorrichtung nach Anspruch 1,
**dadurch gekennzeichnet, daß**
in den Behältern (6) schwammartige Gewebe, insbesondere saugfähige Vliese (18), angeordnet sind.

3. Vorrichtung nach Anspruch 1,
**dadurch gekennzeichnet, daß**
in den Behältern (6) mit trockenen Wirkstoffen versehene Spitzen (8) angeordnet sind.

4. Vorrichtung nach einem der Ansprüche 1 bis 3,
**dadurch gekennzeichnet, daß**
die Behältereinheit (5) aus einer Hohl- oder Doppelfolie besteht, bei der zwei im wesentlichen parallel verlaufende Folien in der Aufsicht netzartig miteinander verschweißt sind, so daß innerhalb der Verschweißungsstege abgeschlossene Hohlräume zwischen den beiden Folien entstehen.

5. Vorrichtung nach einem der Ansprüche 1 bis 3,
**dadurch gekennzeichnet, daß**
- die Behältereinheit (5) eine Blisterpackung ist mit einem Basiskörper (16), welcher einseitig zur gleichen Seite hin offene Vertiefungen (17) aufweist, mit
- einer Schutzfolie (10) zum Abdecken und Verschließen der Vertiefungen (17) und
- saugfähigem Vlies (18) in den Vertiefungen (17), welches mit den Wirkstoffen getränkt ist.

6. Vorrichtung nach Anspruch 5,
**dadurch gekennzeichnet, daß**
in den Vertiefungen (17) der Behälter (6) Spitzen (8) in Richtung der offenen Seiten der Behälter (6) hin gerichtet angeordnet sind, die im Normalzustand der Vertiefungen die freie Außenfläche der Behälter (6) nicht erreichen.

7. Vorrichtung nach Anspruch 3 oder 6,
**dadurch gekennzeichnet, daß**
die Spitzen (8) in analoger Anordnung zu den Behältern (6) der Behältereinheit (5) auf einer Verletzungseinheit angeordnet sind, die elastisch mit der Behältereinheit verbunden ist, so daß beim Zusammenklappen die Spitzen (8) in die Behälter (6) eindringen.

8. Vorrichtung nach einem der vorangehenden Ansprüche,
**dadurch gekennzeichnet, daß**
wenigstens die Abdeckung (2) mittels Klettbändern (20), die mit jeweils zwei freien Enden von der Abdeckung (2) abstreben, an der zu bearbeitenden Oberfläche (1) befestigt werden kann.

9. Vorrichtung nach einem der vorangehenden Ansprüche,
**dadurch gekennzeichnet, daß**
die auf die Oberfläche (1) aufzulegende Abdeckung (2) aus Kunststoff bei jedem als Arbeitsfeld (4) fungierendem Durchbruch (3) eine Kennzeichnung, z. B. eine Numerierung, auf der der Oberfläche (1) zuzuwendenden Seite aufweist, die beim Auflegen auf die Oberfläche (1) auf diese übergeht.

## Claims

1. A device for partially characterising, piercing and wetting a surface when conducting an allergy test comprising
- a cover (2) made from plastic in which parallel rows of adjacent orifices (3) are arranged, and
- a container unit (5), the base surface of which essentially matches that of the cover (2) and which has a substantially flat underside,
- containers (6) corresponding to the orifices (3) being provided in the container unit (5), all of which are open to the same side at one end and serve respectively as receptacles for agents,
characterised in that
- the cover (2) and the container unit (5) are joined to one another at one edge (12) so as to fold so that the containers (6) of the container unit (5) can be placed flush on top of the orifices (3) of the cover (2), and
- the surface can be wetted by folding the cover (2) and the container unit (5) together.

2. A device as claimed in claim 1, characterised in that sponge-like fabrics, in particular absorbent fleeces (18), are arranged in the containers (6).

3. A device as claimed in claim 1, characterised in that spikes (8) provided with dry agents are arranged in the containers (6).

4. A device as claimed in any one of claims 1 to 3, characterised in that the container unit (5) consists of a hollow or two-ply film, in which two films extending substantially parallel are fused to one another in a network design as viewed from above so that sealed hollow cavities are formed between the two films inside the fused lands.

5. A device as claimed in any one of claims 1 to 3, characterised in that - the container unit (5) is a blister pack with a base body (16) having recesses (17) opening to the same side on one side, with
- a protective film (10) for covering and sealing the recesses (17), and
- an absorbent fleece (18) in the recesses (17) which is soaked with the agents.

6. A device as claimed in claim 5, characterised in that spikes (8) are arranged in the recesses (17) of the containers (6) pointing in a direction towards the open ends of the containers (6), which do not reach the free external surface of the containers (6) when the recesses are in their normal state.

7. A device as claimed in claim 3 or claim 6, characterised in that the spikes (8) are arranged in a layout matching the containers (6) of the container unit (5) on a breaking unit which is resiliently joined to the container unit so that the spikes (8) pierce the containers (6) on folding.

8. A device as claimed in any one of the preceding claims, characterised in that at least the cover (2) can be secured to the surface (1) to be worked on by means of adhesive strips (20) which hold the cover (2) down by means of two respective free ends.

9. A device as claimed in any one of the preceding claims, characterised in that on the side to be applied to the surface (1), the plastic cover (2) to be laid on the surface (1) has a characterising mark, e.g. a number, for each orifice (3) serving as a working area (4), which is transferred to the surface (1) when laid thereon.

## Revendications

1. Dispositif pour identifier, léser et mouiller partiellement une surface lors d'un test d'allergie, comprenant
- une partie de recouvrement (2) en matière plastique dans laquelle des ouvertures (3) sont disposées les unes à côté des autres, en rangées sensiblement parallèles, ainsi
- qu'une unité réceptrice (5) dont la surface de base correspond essentiellement à celle de la partie de recouvrement (2) et qui présente une face inféricure essentiellement plane,
- des alvéoles (6) correspondant aux ouvertures (3) étant prévus dans l'unité réceptrice (5), lesquels alvéoles sont tous ouverts d'un côté, en direction du même côté, et ont une fonction de réservoir pour des substances actives,
caractérisé par le fait que
- la partie de recouvrement (2) et l'unité réceptrice (5) sont liées l'une à l'autre avec possibilité de pliage au d'un bord, de telle manière que les alvéoles (6) de l'unité réceptrice (5) puissent être amenés en coïncidence avec les ouvertures (3) de la partie de recouvrement (2) et que
- la surface peut être mouillée en repliant l'une sur l'autre la partie de recouvrement (2) et l'unité réceptrice (5).

2. Dispositif selon la revendication 1, caractérisé par le fait que des tissus de type éponge, en particulier des feutres (6) absorbants, sont disposés dans les alvéoles (6).

3. Dispositif selon la revendication 1, caractérisé par le fait que des pointes (8) ponant des substances actives sèches sont disposées dans les alvéoles (6).

4. Dispositif selon une des revendications 1 à 3, caractérisé par le fait que l'unité réceptrice (5) est formée d'un film creux ou d'un film double , dans lequel deux films essentiellement parallèles sont soudés l'un à l'autre en une sorte de réseau en vue de dessus, de manière à former des cavités fermées entre les cordons de soudure, entre les deux films.

5. Dispositif selon une des revendications 1 à 4, caractérisé par le fait que
- l'unité réceptrice (5) est un emballage de type blister avec un corps de base (16) qui comporte des cavités (17) ouvertes d'un côté, on direction du même côté, avec
- un film de protection (10) pour couvrir et fermer les cavités (17) et
- un feutre (18) absorbant placé dans les cavités (17), qui est imprégné des substances actives.

6. Dispositif selon la revendication 5, caractérisé par le fait que des pointes (8) dirigées vers les côtés ouverts des alvéoles (6) sont disposées dans les cavités (17) des alvéoles (6), lesquelles pointes, en position normale, n'atteignent pas la surface extérieure des alvéoles (6).

7. Dispositif selon une des revendications 3 ou 6, caractérisé par le fait que les pointes (8) sont disposées de manière analogue aux alvéoles (6) dans l'unité réceptrice (5) sur une unité d'incision qui est liée de manière élastique à l'unité réceptrice de manière à ce que les pointes (8) pénètrent dans les alvéoles (6) lors du pliage.

8. Dispositif selon une des revendications précédentes, caractérisé par le fait qu'au moins la partie de recouvrement (2) peut être fixée à la surface (1) à traiter au moyen de rubans auto-agrippants (20) dont les deux extrémités s'étendent au-delà chaque fois de la partie de recouvrement (2).

9. Dispositif selon une des revendications précédentes, caractérisé par le fait que la partie de recouvrement (2) en matière plastique à appliquer sur la surface (1), pour chaque ouverture (3) qui représente une zone de traitement (4), comporte une identification, par exemple un numéro, sur sa face tournée vers la surface (1), laquelle identification est transférée sur la surface (1) lors de l'application.
